# NOUVEAU FASCICULE DE BREVET EUROPEEN

(11) **EP 0 353 108 B2**
(45) Date de publication et mention de la décision concernant l'opposition: **17.09.2003**
(45) Mention de la délivrance du brevet: 18.05.1994
(21) Numéro de dépôt: 89401599.9
(22) Date de dépôt: 09.06.1989
(51) Int. Cl.: A61K 39/12

(54) **Procédé de stabilisation des vaccins et associations de vaccins à virus atténués conservés sous forme lyophilisée, et compositions obtenues**
Verfahren zur Stabilisierung von Impfstoffen, Assoziationen von attenuierten, lyophilisierten Impfstoffen und erhaltene Zusammensetzungen
Process for stabilizing vaccines and associations of attenuated lyophilized viral vaccines, and compositions obtained

(30) Priorité: 30.06.1988 FR 8808806
(43) Date de publication de la demande: 31.01.1990
(73) Titulaire: Aventis Pasteur, 69367 Lyon Cedex 07 (FR)
(72) Inventeur: Françon, Alain Jean-Louis, F-69690 Bessenay (FR); Montagnon, Bernard Jean, F-69210 L'Arbresle (FR)
(74) Mandataire: Bernasconi, Jean

(56) Documents cités:
- EP-A- 0 252 059
- DE-A- 1 944 956
- Package insert for IB vaccin NOBILIS D274 (version 11.09.86.) mentioning thioreum
- Package insert for IB vaccin NOBILIS D274 (version 27.02.86.) mentioning thioreum
- Price list of products offered for sale by Intervet NV, dated 01.10.87, listing IB vaccin D274
- Registration Documents D274, 27.02.86.

## Description

L'invention concerne un procédé de stabilisation des vaccins et associations de vaccins à virus atténués qui sont conservés sous forme lyophilisée en présence d'au moins un excipient de lyophilisation.

L'invention concerne également les compositions obtenues par le procédé conforme à l'invention.

De nombreux vaccins contre les maladies virales sont fabriqués à partir de virus vivants atténués. La lyophilisation est utilisée pour leur conservation. Cette technique de dessiccation de produits préalablement congelés s'applique aux vaccins préparés par mélanges appropriés de virus atténués avec des excipients de lyophilisation dont le rôle est de protéger le produit (STONES P.B., WARDEN D. and KERR J. Symposium on stability and effectiveness of measles, poliomyelitis and pertussis vaccines (1976) Zagreb - Yougoslavie. The stability of measles vaccine and factors affecting its efficacy). Dans les associations de vaccins, chaque valence a en général un excipient de lyophilisation qui lui est propre.

Cependant, malgré la lyophilisation, on observe, avec certains vaccins, une perte d'activé sensible du vaccin lyophilisé lorsqu'il est conservé à température ordinaire. On pense que cette perte d'activité serait en partie due à une dégradation des particules virales atténuées, certains virus atténués y étant plus sensibles que d'autres.

La présente invention a pour objectif de remédier à ce problème en fournissant un procédé de stabilisation aboutissant à une meilleure conservation dans le temps des vaccins et associations de vaccins à virus atténués conservés sous forme lyophilisée.

Un objectif de l'invention est de fournir un procédé qui soit simple à mettre en oeuvre, sûr et peu onéreux et qui utilise des produits qu'il est possible d'obtenir en quantités suffisantes et qui soient exempts de toxicité pour l'homme.

L'invention a pour objet un procédé de stabilisation des vaccins et associations de vaccins à virus atténués qui sont conservés sous forme lyophilisée en présence d'au moins un excipient de lyophilisation, caractérisé en ce qu'on ajoute, de préférence avant la lyophilisation, une quantité appropriée d'au moins un produit appartenant au groupe constitué par l'urée, l'allylurée, l'acétamide, le méthylcarbamate et le butylcarbamate

Le composé stabilisant selon l'invention ne se substitue pas aux excipients de lyophilisation habituels mais au contraire vient compléter la stabilisation des vaccins et associations de vaccins visés par l'invention,

De préférence, le composé stabilisant est l'urée, qui présente l'avantage d'être un produit physiologique naturellement présent dans l'organisme :

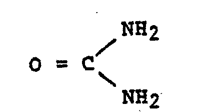

Les autres composés stabilisants selon l'invention ont la formule
- l'allylurée 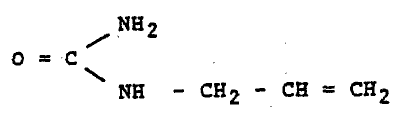
- l'acétamide 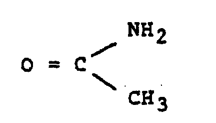
- le méthylcarbamate 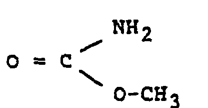
- le butylcarbamate

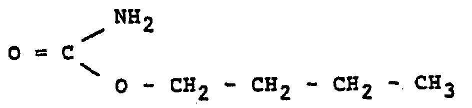

Le produit stabilisant est utilisé à une concentration finale très faible, par exemple de l'ordre de 0,5 % (en poids). Chaque produit peut avoir une plage de concentration plus ou moins étendue, avec un effet stabilisant, et au delà de cette plage, un effet néfaste pour le ou les virus. Par exemple pour l'allylurée, il est préférable de ne pas dépasser 0,5 %.

L'urée peut être utilisée à une concentration finale allant de 0,125 à 2 % environ, étant entendu que cette plage de concentration préférée n'est pas limitative.

On préfère introduire le composé stabilisant avant la lyophilisation. Toutefois, l'action du stabilisant peut être démontrée, même si on l'introduit après cette dernière (réhydratation et nouvelle lyophilisation).

L'étude détaillée qui va suivre prend comme exemple le vaccin triple rougeole, oreillons, rubéole dans lequel les valences rougeole et oreillons sont sujettes à des pertes d'activité importantes. Mais l'invention concerne tous les vaccins à virus atténués recontrant des difficultés de conservation à l'état lyophilisé pour des raisons similaires, telles que la présence de neuraminidase et/ou de glycoprotéines sensibles.

Le tableau I retrace les résultats d'une étude de l'action de l'urée sur des lots de vaccin industriels.

**TABLEAU I**

| Comparaison des pertes du titre infectieux en 7 jours à + 37 C, sur des lots industriels, avec et sans Urée. | | | | | |
|---|---|---|---|---|---|
| **VALENCE** | UREE % | LOT | TITRES EN DICc50/DOSE | | PERTE MOYENNE (log) * |
| | | | - 20°C | 7 jours à + 37°C | |
| **ROUGEOLE** | 0 | A 1264 | 3,93 | 2,73 | 0,86 |
| | | A 1265 | 3,86 | 2,97 | |
| | | A 1266 | 4,03 | 3,53 | |
| | 0,5 | B 1193 | 3,68 | 3,00 | 0,61 |
| | | B 1331 | 4,13 | 3,53 | |
| | | B 1379 | 3,98 | 3,43 | |
| **OREILLONS** | 0 | A 1264 | 4,45 | 3,23 | 1,34 |
| | | A 1265 | 4,40 | 3,00 | |
| | | A 1266 | 4,48 | 3,07 | |
| | 0,5 | B 1193 | 4,36 | 3,80 | 0,55 |
| | | B 1331 | 4,37 | 3,80 | |
| | | B 1379 | 4,59 | 4,07 | |
| **RUBEOLE** | 0 | A 1264 | 3,58 | 3,13 | 0,38 |
| | | A 1265 | 3,72 | 3,30 | |
| | | A 1266 | 3,57 | 3,30 | |
| | 0,5 | B 1193 | 3,49 | 3,20 | 0,34 |
| | | B 1331 | 3,43 | 3,17 | |
| | | B 1379 | 3,68 | 3,20 | |

| | | | | | |
|---|---|---|---|---|---|
| * Logarithme base 10 | | | | | |

Pour chacune des valences du vaccin triple, on a obtenu une stabilité supérieure à celle obtenue uniquement avec les mêmes excipients de lyophilisation mais sans adjonction d'urée, en particulier après dégradation d'une semaine à 37°C, excepté pour la valence rubéole dont la stabilité est déjà assurée de façon satisfaisante.

L'invention va être maintenant décrite plus en détail à l'aide d'exemples non limitatifs s'appliquant au vaccin triple rougeole, oreillons, rubéole.

Pour ces exemples, le mode opératoire est identique :

Les produits viraux concentrés rougeole, oreillons et rubéole, conservés congelés à basse température, sont décongelés puis mélangés extemporanément. Ce mélange est ensuite réparti entre les différents essais, avec l'excipient de lyophilisation additionné ou non de produit stabilisant selon l'invention. On procède ainsi de façon à obtenir les proportions de solutions finales suivantes rougeole : 1,5 volume ; oreillons : 0,01 volume ; rubéole 1,0 volume et excipient 2,5 volumes.

Ces différentes proportions, indiquées par rapport aux exemples, peuvent varier, selon le titre des produits viraux concentrés et ne sont pas limitatives.

Les essais sont répartis en petits flacons puis lyophilisés suivant un cycle standard. Les tests de stabilité sont faits en plaçant des flacons pendant 7 jours à +37°C ou 6 jours à + 45°C, avec des témoins à + 4°C. Ces flacons sont ensuite réhydratés et titrés. L'activité est déterminée par le nombre de doses infectieuses à 50 % en culture de tissu (DICC50). Pour la méthode des dilutions limites, sur microplaques, cela correspond à la dilution théorique calculée, pour laquelle la moitié des cupules ensemencées présente un effet cytopathique caractéristique.

Les cellules utilisées pour les titrages des valences rougeole et oreillons sont des cellules Vero et, pour la valence rubéole, des cellules RK13. Pour chaque valeur indiquée dans les tableaux, le chiffre de'titre est une moyenne de quatre titrages différents, au minimum.

### Exemple 1 : Effet de l'urée sur le vaccin rougeole, oreillons, rubéole, en présence de différents excipients de lyophilisation.

Les différents excipients de lyophilisation utilisés ont une composition classique pour la lyophilisation (produits dits de charge, cryoprotecteurs, etc.). Leur composition diffère au niveau des sucres et des acides aminés, ce qui est suffisant pour modifier la stabilité des différentes valences, en l'absence de produit stabilisant.

Les différents titrages de cet exemple ont été réalisés par essais. Un témoin commun (T) permet de comparer les différentes valeurs obtenues. Les pertes d'activité en 7 jours à + 37°C ou en 6 jours à + 45°C, permettent de juger de la stabilité de chaque valence en fonction de l'excipient de lyophilisation utilisé, ainsi que de l'effet stabilisant de l'urée pour chacun d'eux.

### TABLEAUX A.

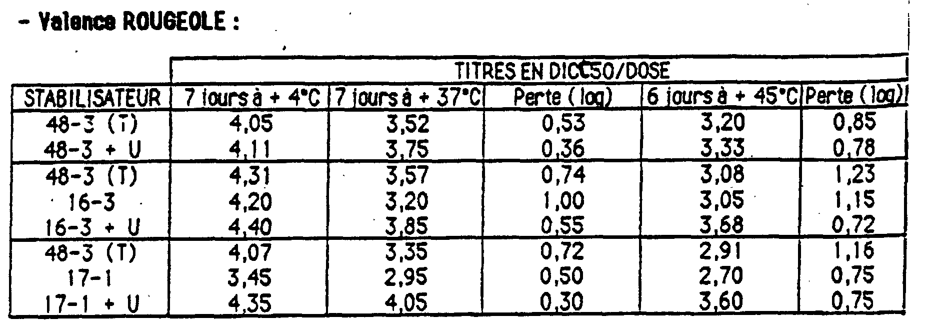

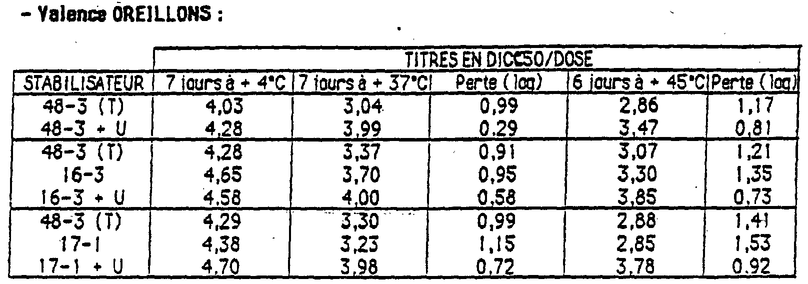

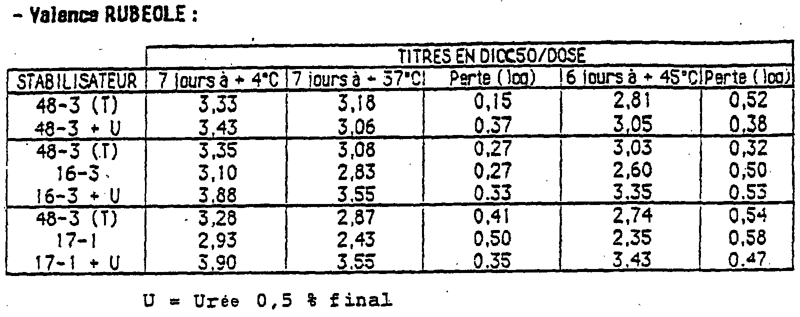

Cet effet stabilisant est essentiellement net pour les valences rougeole et oreillons.

La valence rubéole est relativement stable avec les trois excipients de lyophilisation testés.

### Exemple 2 : Effet stabilisant de plusieurs produits proches.

L'urée, et l'allylurée ont été testés avec le même excipient de lyophilisation et pour les trois valences du vaccin.

L'acétamide, le méthylcarbamate et le butylcarbamate ont été testés de la même manière uniquement par rapport à la valence oreillons qui est la plus instable.

Les différents essais ont été réalisés pour des vaccins préparés en excipient sans produit stabilisant selon l'invention (Témoin) et avec produit stabilisant selon l'invention (produits étudiés). Les titrages ont été réalisés sur ces différents produits.

Dans cet exemple, les produits sont tous utilisés à la même concentration finale : 0,5 %.

### TABLEAUX B.

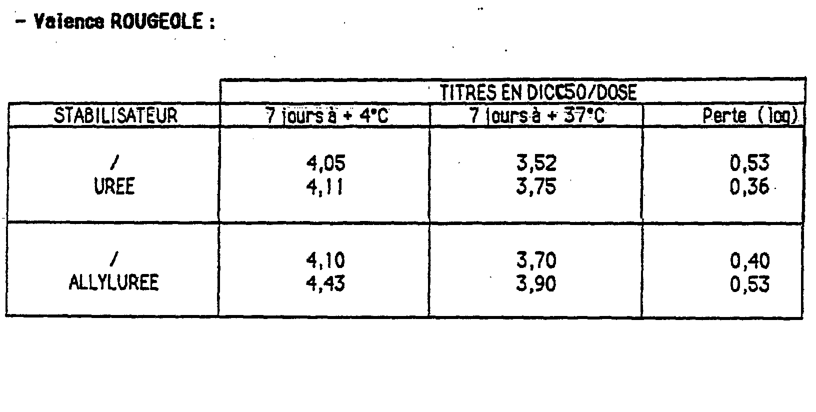

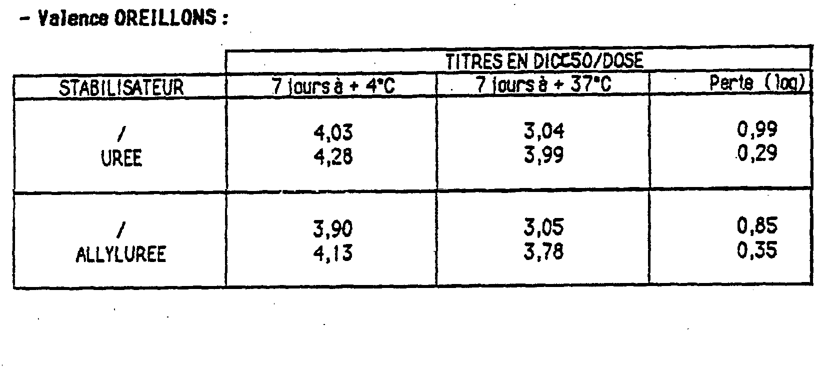

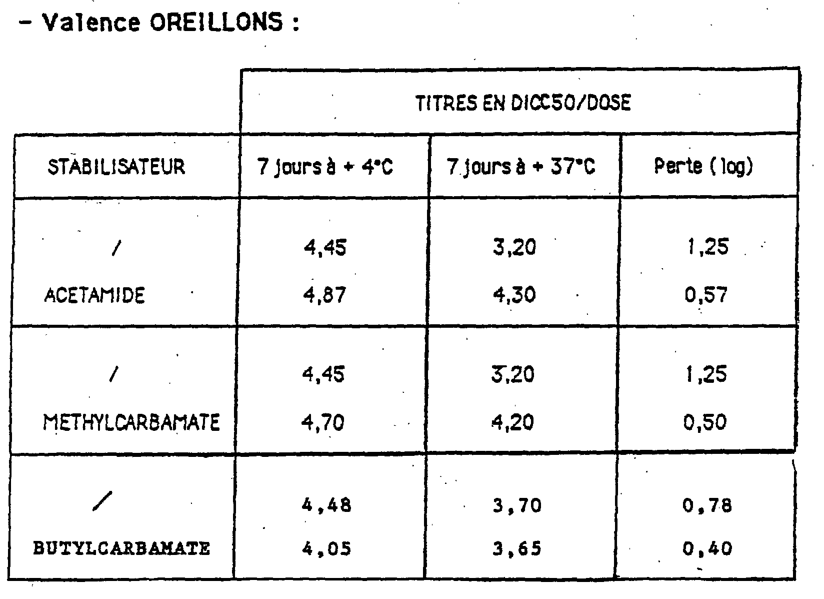

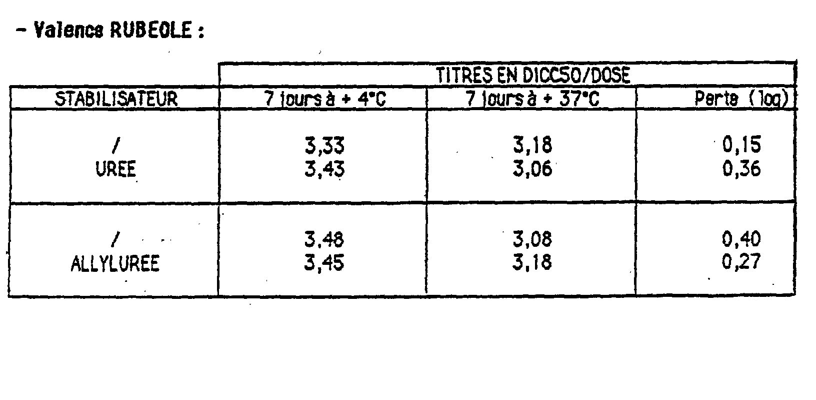

L'effet stabilisant de ces produits est proche et se manifeste surtout au niveau de la valence oreillons, avec l'excipient de lyophilisation utilisé.

Dans ces exemples, la composition détaillée, non critique, des excipients de stabilisation 48-3, 16-3 et 17-1 est la suivante :

### 48-3

Lactose (D+), milieu de culture, albumine humaine, acide glutamique, phosphate monopotassique, phosphate bipotassique, potasse, arginine, chlorhydrate, cystine, histidine, isoleucine, leucine, chlorhydrate de lysine, méthionine, phenylalanine, thréonine, tryptophane, tyrosine, valine, alanine, asparagine, acide aspartique, glycine, proline, sérine, sorbitol (D-), mannitol (D-), dextran 70
à des concentrations respectives de
2-7 mg ; 0.4 à 1.5 mg ; 0.8 à 3 mg ; 0.05 à 0.15 mg ; 0.01 à 0.05 ; 0.05 à 0.2 mg ; 0.008 à 0.05 mg ; 2.4 à 6 mg ; 0.02 à 0.06 mg ; 0.018 à 0.05 mg ; 0.05 à 0.2 mg ; 0.05 à 0.2 mg ; 0.08 à 0.20 mg ; 0.01 à 0.05 mg ; 0.04 à 0.10 mg ; 0.05 à 0.15 mg ; 0.008 à 0.02 mg ; 0.03 à 0.10 mg ; 0.05 à 0.12 mg ; 0.015 à 0.05 mg ; 0.02 à 0.08 mg ; 0.02 à 0.08 mg ; 0.015 à 0.05 mg ; 0.02 à 0.06 mg ; 0.02 à 0.05 mg ; 5 à 15 mg ; 2 à 8 mg ; 1.5 à 4 mg ; par dose de produit final.

### 16-3

Même formule que 48-3, mais sans mannitol.

### 17-1

Même formule que 48-3, mais avec proportions différentes de certains acides aminés et sucres.

### Exemple 3 : Effet de la concentration en produit stabilisant sur la stabilité du vaccin triple.

Cette étude a été réalisée avec l'urée et l'allylurée, sur les trois valences et en présence de l'excipient de lyophilisation de l'exemple 2, pour des concentrations en produit selon l'invention variant de 0,125 à 2,0 % en final.

### TABLEAUX C.

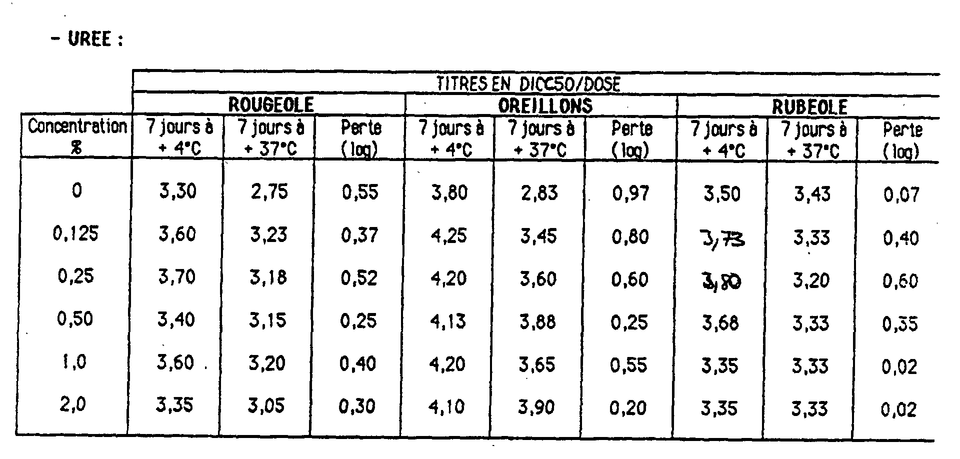

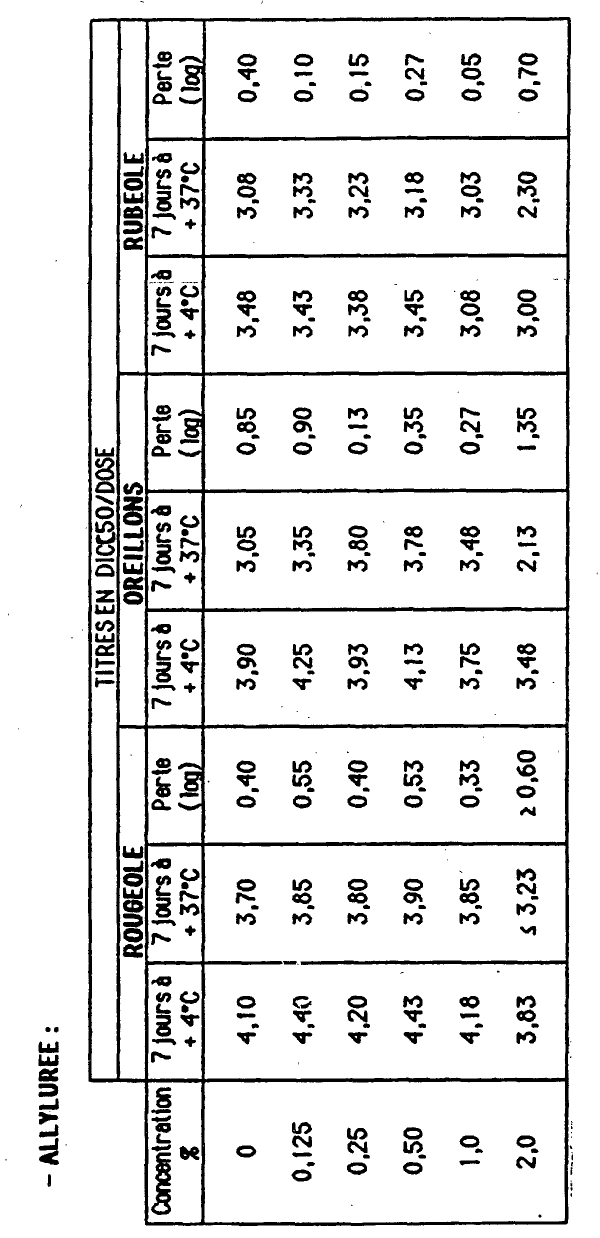

Pour l'urée, la stabilité du vaccin augmente avec la concentration du produit jusqu'à 0,5 % puis, au-delà, la stabilité ne change pas.

Avec l'allylurée, on obtient la même progression de la stabilité du vaccin jusqu'à 0,5 % puis, au-delà, une diminution allant jusqu'à une stabilité inférieure à celle du témoin sans allyfurée.

Pour cet exemple, les essais et les titrages ont été regroupés par produit, pour leur réalisation, ce qui permet de comparer le niveau des titres à + 4°C, + 37 °C, ainsi que les pertes, obtenus pour un même produit.

### Exemple 4 : Effet de l'urée sur des vaccins rougeole, oreillons, rubéole lyophilisés, réhydratés en présence ou non d'urée, puis relyophilisés.

L'étude a été élargie à des vaccins du commerce. Ces vaccins, nommés A, B, et C, n'ont pas tous la même souche de virus ourlien.

Ces vaccins ont été réhydratés soit avec de l'eau, soit avec une solution d'urée à 0,5 % puis relyophilisés selon un cycle standard. Ces vaccins ont été traités dans les mêmes conditions, avec une référence (vaccin de la déposante, I.M.) étudiée en présence ou non d'urée au moment de la première lyophilisation et au moment de la deuxième lyophilisation.

L'étude a été effectuée uniquement sur la valence oreillons qui est la plus instable.

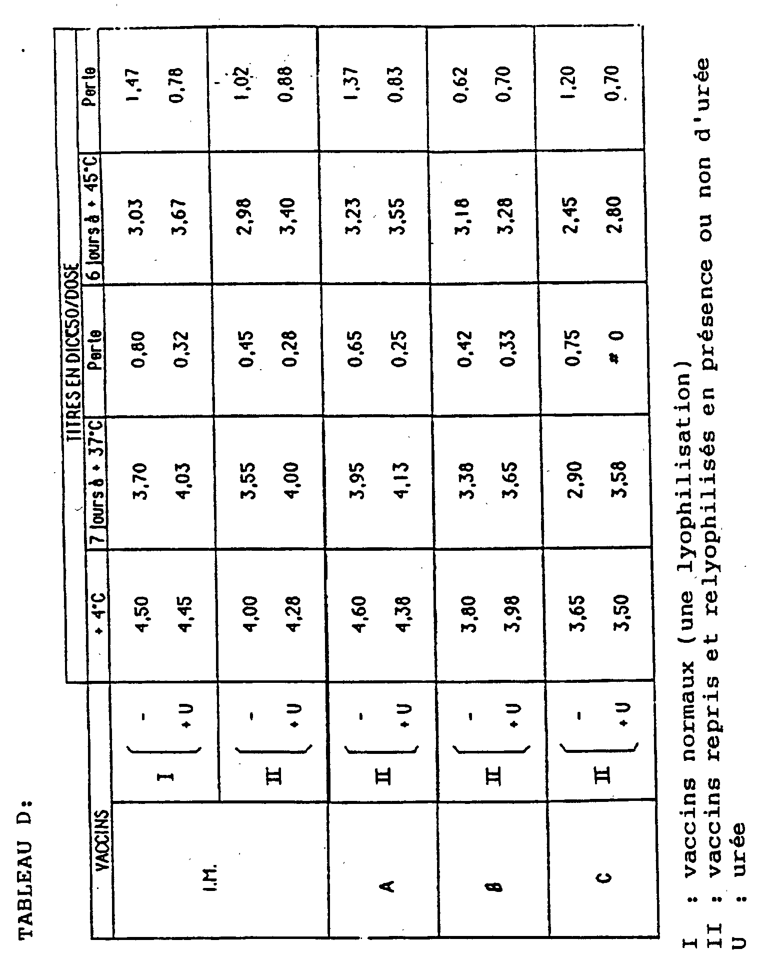

Comme il ressort du tableau D, l'urée améliore la stabilité de la valence oreillons dans les vaccins I.M., A et C. Pour le vaccin B, la forte stabilité observée après la deuxième lyophilisation ne permet pas de mettre en évidence l'action de l'urée.

## Revendications

1. Procédé de stabilisation des vaccins et associations de vaccins à virus atténués, notamment rougeole, rubéole, virus ourlien, qui sont conservés sous forme lyophilisée en présence d'au moins un excipient de lyophilisation, **caractérisé en ce qu'**on ajoute, de préférence avant la lyophilisation, une quantité appropriée d'au moins un produit appartenant au groupe constitué par l'urée, l'allylurée, l'acétamide, le methylcarbamate et le butylcarbamate

2. Procédé selon la revendication 1, **caractérisé en ce que** le produit est utilisé à une concentration finale de l'ordre de 0,5 %.

3. Procédé selon la revendication 1, **caractérisé en ce que** l'urée est utilisée à une concentration finale allant de 0,125 à 2% environ.

## Patentansprüche

1. Verfahren zur Stabilisierung von Impfstoffen und Kombinationen von Impfstoffen mit abgeschwächten Viren, insbesondere dem Masern-, Röteln-, Mumps-Virus, die in lyophilisierter Form in Gegenwart mindestens eines Lyophilisierungs-Hilfstoffes konserviert sind,
**dadurch gekennzeichnet,**
**daß** man, vorzugsweise vor der Lyophilisierung, eine geeignete Menge mindestens eines Produkts zugibt, das zu der Gruppe gehört, die aus Harnstoff, Allylharnstoff, Acetamid, Methylcarbonat und Butylcarbonat besteht.

2. Verfahren nach Anspruch 1,
**dadurch gekennzeichnet,**
**daß** das Produkt in einer Endkonzentration in der Größenordnung von 0,5 % verwendet wird.

3. Verfahren nach Anspruch 1,
**dadurch gekennzeichnet,**
**daß** der Harnstoff in einer Endkonzentration, die von ungefähr 0,125 bis 2 % geht, verwendet wird.

## Claims

1. Process for stabilizing attenuated virus vaccines and combinations of vaccines, especially measles, rubella and mumps virus, that are preserved in lyophilised form in the presence of at least one lyophilisation excipient, **characterized in that**, preferably before lyophilisation, a suitable amount at least one product belonging to the group composed of urea, alkylurea, acetamide, methylcarbamate and butylcarbamate, is added.

2. Process according to claim 1, **characterized in that** the product is used at a final concentration of the order of 0.5%.

3. Process according to claim 1, **characterized in that** urea is used at a final concentration ranging from 0.125 to 2% approximately.
